# EUROPEAN PATENT APPLICATION

(11) **EP 4 564 008 A1**
(43) Date of publication of application: **04.06.2025**
(21) Application number: 23845967.1
(22) Date of filing: 19.05.2023
(51) Int. Cl.: G01N 35/00

(54) **AUTOMATIC ANALYSIS SYSTEM**

(30) Priority: 27.07.2022 JP 2022119510
(71) Applicant: SHIMADZU CORPORATION, Kyoto-shi, Kyoto 604-8511 (JP)
(72) Inventor: SUGIYAMA, Kiyohiro, Kyoto-shi, Kyoto 604-8511 (JP)
(74) Representative: Müller-Boré & Partner Patentanwälte PartG mbB
(86) International application number: PCT/JP2023/018822
(87) International publication number: WO 2024/024229

(57) **Abstract**

An embodiment includes: an analysis part (10) configured to be able to selectively perform a general sample analysis and a quality control analysis; a display input part (30); a timing setting storage part (40) which stores a setting regarding an execution timing of the quality control analysis; a display control part (50) configured to display a deferment instruction key on the display input part at a predetermined timing before the execution timing determined based on the setting stored in the timing setting storage part; and a deferment control part (60) configured to defer the execution timing based on a fact that a predetermined operation is performed on the deferment instruction key. As a result, in an automatic analysis device configured to perform the quality control analysis at the preset timing, appropriate analysis control depending on the situation can be performed.

## Description

### TECHNICAL FIELD

The present invention relates to an automatic analysis system capable of continuously and automatically analyzing a plurality of samples.

### BACKGROUND ART

Conventionally, an automatic analysis device that automatically performs quantitative analysis for a specific component contained in a sample, such as blood or urine, is known. In such an automatic analysis device, a mixed solution obtained by mixing a reagent corresponding to a specific component (hereinafter, the component is referred to as inspection item component) and a sample is irradiated with light, and absorbance or turbidity (scattered light intensity or transmitted light intensity) of the mixed solution is measured. When the inspection item component is contained in the sample, the inspection item component and the reagent react with each other, and the absorbance or turbidity of the mixed solution changes depending on the concentration of the inspection item component. Therefore, by preparing in advance a calibration curve indicating the relationship between the concentration and the absorbance or turbidity of the inspection item component, the concentration of the inspection item component in the sample can be calculated based on the calibration curve.

In the automatic analysis device, quality control analysis is usually performed before the start of an analysis such as when the power is turned on, or every time a predetermined time elapses from the start of first analyses, or every time a predetermined number of samples are analyzed. In the quality control analysis, the above-described measurement is performed on a quality control sample containing an inspection item component having a known concentration to obtain the concentration of the inspection item component, and the quality control of quantitative analysis is performed by checking whether or not the obtained concentration is within a prescribed range (see, for example, Patent Literature 1).

### CITATION LIST

### PATENT LITERATURE

Patent Literature 1: JP 2010-151707 A

### SUMMARY OF INVENTION

### TECHNICAL PROBLEM

In a case where the quality control analysis is performed at preset times during analyses, such as every time a predetermined time elapses or every time a predetermined number of samples are analyzed, some equipment settings may limit the subsequent analysis of samples (hereinafter referred to as "general sample analysis") unless a quality control analysis is performed. Such restrictions are inconvenient, for example, when there are samples whose analysis results must be reported in a hurry, or when general sample analysis is delayed due to a large number of unanalyzed samples.

In addition, there is a case where it can be determined, from results of the past quality control analysis and results of the past general sample analysis, that there is no problem even if the quality control analysis is not immediately performed. When there are only a few unanalyzed samples left, it may be more convenient to continue general sample analysis without performing quality control analysis.

The present invention has been made in view of the above problems, and an object of the present invention is to enable appropriate analysis control depending on a situation in an automatic analysis device which performs quality control analysis at a preset time.

### SOLUTION TO PROBLEM

An automatic analysis system according to a first aspect of the present invention made to solve the above problems includes:
an analysis part configured to be able to selectively perform a general sample analysis and a quality control analysis;
a display input part;
a timing setting storage part which stores a setting regarding an execution timing of the quality control analysis;
a display control part configured to display a deferment instruction key on the display input part at a predetermined timing before the execution timing determined based on the setting stored in the timing setting storage part; and
a deferment control part configured to defer the execution timing based on the fact that a predetermined operation is performed on the deferment instruction key.

A display processing device for an automatic analysis system according to a second aspect of the present invention made to solve the above problems is a display processing device for the automatic analysis system equipped with an analysis part configured to be able to selectively perform a general sample analysis and a quality control analysis, including:
a display input part;
a timing setting storage part which stores a setting regarding an execution timing of the quality control analysis;
a display control part configured to display a deferment instruction key on the display input part at a predetermined timing before the execution timing determined based on the setting stored in the timing setting storage part; and
a deferment control part configured to defer the execution timing based on the fact that a predetermined operation is performed on the deferment instruction key.

### ADVANTAGEOUS EFFECTS OF INVENTION

According to the present invention, the user can defer the timing of a quality control analysis with a simple operation, and can perform an analysis control appropriate to given analysis situation.

### BRIEF DESCRIPTION OF DRAWINGS

[Fig. 1] A configuration diagram schematically illustrating an automatic analysis system according to an embodiment of the present invention.
[Fig. 2] A diagram illustrating an example of a screen on which a deferment instruction key is displayed in the embodiment.
[Fig. 3] A diagram illustrating an example of a screen on which an analysis result is displayed in the embodiment.
[Fig. 4] A flowchart illustrating a procedure of a processing of a display control part in the embodiment.
[Fig. 5] A flowchart illustrating a procedure of a processing of a deferment control part in the embodiment.
[Fig. 6] A flowchart illustrating a procedure of a processing of a quantitation part in the embodiment.
[Fig. 7] A flowchart illustrating a procedure of a processing of an output part in the embodiment.

### DESCRIPTION OF EMBODIMENTS

Hereinafter, an embodiment of an automatic analysis system according to the present invention will be described with reference to the drawings. The automatic analysis system according to the present invention is an analysis system which can be used for a device capable of automatically and continuously analyzing a plurality of samples, such as a biochemical automatic analysis device, an immunity item analysis device, or a blood coagulation automatic analysis device. Note that, in the present embodiment, it is assumed that quality control analysis is not automatically performed but is performed when a user performs a predetermined operation.

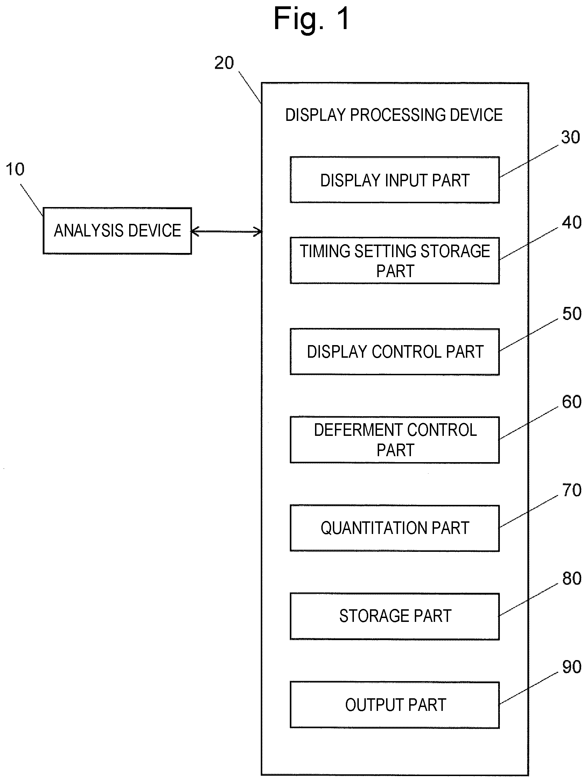

Fig. 1 is a configuration diagram schematically illustrating an automatic analysis system 1. The automatic analysis system 1 includes an analysis device 10 (analysis part in the present invention) configured to analyze a sample and a display processing device 20 configured to display information regarding the analysis device 10.

The analysis device 10 is configured to perform a predetermined measurement on a general sample which is a sample and an accuracy control sample including an inspection item component of a known concentration. In the present embodiment, the analysis device 10 includes a light source and a photodetector, and irradiates a mixed solution obtained by mixing a reagent corresponding to an inspection item component and a sample with light from the light source, to measure a change in light intensity (absorbance or turbidity) detected by the photodetector. The analysis device 10 can analyze a plurality of inspection item components for one sample. For example, one sample is dispensed into a plurality of containers, mixed with a reagent corresponding to the inspection item component in each container, and the above measurement is performed on the mixed solution. The analysis device 10 according to the present embodiment is set so that the subsequent general sample analysis cannot be performed unless the quality control analysis is normally executed.

The display processing device 20 includes a display input part 30, a timing setting storage part 40, a display control part 50, and a deferment control part 60. The entity of the display processing device 20 is, for example, a general-purpose personal computer (PC), which can exert the function of each configuration (the display input part 30, the timing setting storage part 40, the display control part 50, the deferment control part 60, and a quantitation part 70, a storage part 80, and an output part 90 to be described later) by operating on the PC the dedicated control and processing software installed in the PC. The display processing device 20 is connected to the analysis device 10 via a network cable or a wireless LAN.

The display input part 30 includes, for example, a display such as a liquid crystal display, and an operation input unit such as a mouse, a touch panel, and a numeric keypad. The display input part 30 displays a plurality of screens including various types of information such as analysis conditions, analysis progress, and analysis results in the analysis device 10, and screens can be switched by selecting keys and tabs drawn on the screens with a mouse, a touch panel, or the like.

The timing setting storage part 40 stores a setting related to the execution timing of the quality control analysis. The execution timing of the quality control analysis may be set depending on the date and time, or may be set depending on the interval (execution interval) of the quality control analysis performed continuously and the starting date and time. The starting date and time is, for example, the date and time when the analysis device 10 is powered on, the date and time when the analysis work is started, the date and time when the quality control analysis is executed, and the like. The interval may be an interval of time such as every 2 hours, or may be an interval of the number of samples such as every 50 samples. In addition, the interval may be set in both the time and the number of samples, and each interval length may change. In the reagent used for the quality control analysis, a recommended interval of the quality control analysis is set depending on the stability. Therefore, when the user sets the interval on the setting screen (not illustrated) displayed on the display input part 30 depending on the analysis situation while referring to the recommended interval, the execution timing may be set accordingly and stored in the timing setting storage part 40. When the recommended interval for each type of reagent is stored in advance in the timing setting storage part 40 (or another storage part) and the type of reagent or the inspection item is selected, the execution timing of the quality control analysis may be set accordingly and stored in the timing setting storage part 40.

The display control part 50 monitors the elapsed time from the starting date and time or the number of general samples measured by the analysis device 10, and displays a deferment instruction key for deferring the execution timing of the quality control analysis on the display input part 30 at a predetermined timing before the execution timing of the next quality control analysis. Note that "display" includes enabling a key already displayed from a state in which the key cannot be operated to a state in which the key can be operated. The timing to display the deferment instruction key may be immediately before the execution timing of the next quality control analysis, but preferably a timing at which about 50 to 90% of the interval from the starting date and time to the execution timing of the next quality control analysis has elapsed.

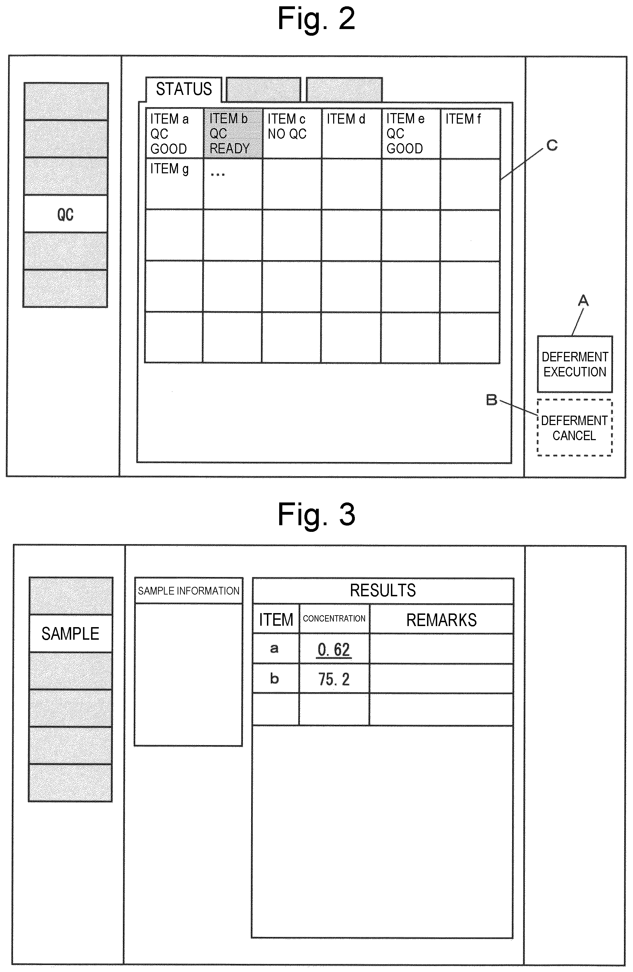

Fig. 2 is an example of a screen of the display input part 30 on which the deferment instruction key is displayed. In Fig. 2, a deferment instruction key A is displayed on a screen indicating the execution status (hereinafter referred to as status information) of the quality control analysis for each inspection item. The status information means letters such as, for example, "QC good" (QC is an abbreviation of Quality Control) when the concentration of the inspection item component in the accuracy control sample is within a prescribed range as a result of performing the quality control analysis, "QC abnormal" when the concentration is not within the prescribed range, "QC failure" when the quality control analysis is not normally performed, and "No QC" when the quality control analysis is not performed in the middle of the general sample analysis, or means icons corresponding to the above letters. In Fig. 2, each cell in Table C corresponds to each inspection item, and the status information in each inspection item can be confirmed. For example, "QC good" is displayed in the cell (Item a) of the first row from the top and the first column from the left in Table C, indicating that the quality control analysis has been normally performed for the item a and the accuracy control is in a good state. "No QC" is displayed in the cell (Item c) in the first row from the top and the third column from the left in Table C, indicating that the quality control analysis in the middle of the analysis is not performed for the Item c. Note that Fig. 2 illustrates a state in which Item b has been selected.

The display control part 50 not only displays the deferment instruction key A at a predetermined timing before the execution timing of the quality control analysis, but also updates the status information displayed on the display input part 30 at the predetermined timing before the execution timing of the quality control analysis or at the execution timing of the quality control analysis has elapsed. For example, the status information is updated to "QC preparation" at the predetermined timing before the execution timing of the quality control analysis, and the status information is updated to "QC excess" at the timing when the execution timing of the quality control analysis has elapsed. The deferment instruction key A is displayed only during a period when the status information is "QC preparation", and the deferment instruction key A is deleted from the display input part 30 when the status information is changed to "QC excess". Furthermore, as will be described later, the deferment instruction key A is also deleted when a predetermined operation is performed on the deferment instruction key A. Note that "delete" includes disabling a key displayed from a state in which the key can be operated to a state in which the key cannot be operated.

The deferment control part 60 changes the setting stored in the timing setting storage part 40 based on the fact that a predetermined operation (for example, an input operation in which the user clicks the deferment instruction key with a mouse on the display input part 30) is performed on the deferment instruction key A, and defer the execution timing of the quality control analysis. For example, the execution timing is delayed by adding an interval between the execution timing of the quality control analysis scheduled to be deferred and the execution timing of the quality control analysis scheduled to be executed subsequently to an interval between the date and time when the previous quality control analysis is executed and the execution timing of the quality control analysis scheduled to be deferred, or by skipping the execution timing of the quality control analysis scheduled to be deferred. The deferment control part 60 further updates the status information to, for example, "under QC deferment" based on the fact that a predetermined operation is performed on the deferment instruction key A, and displays a deferment cancel key B indicated by a dotted line in Fig. 2 on the display input part 30. At this time, the deferment instruction key A is deleted. In addition, the deferment cancel key B is deleted at the timing when the quality control analysis is executed after the deferment. When the deferment processing continues by the deferment control part 60, the reliability of the accuracy control decreases, and thus, the processing of displaying the deferment instruction key A in the display control part 50 is not performed for the execution timing of the quality control analysis after the deferment.

The display processing device 20 according to the present embodiment further includes a quantitation part 70, a storage part 80, and an output part 90. The quantitation part 70 calculates the concentration of the inspection item component in the general sample based on a calibration curve created in advance, from the measurement data related to the light intensity measured by the analysis device 10. Then, the calculation result is stored in the storage part 80. At this time, a flag is attached to and stored in the calculation result of the general sample analyzed within the period in which the execution timing of the quality control analysis is deferred (until the deferred execution timing of the quality control analysis elapses, after the preset execution timing of the quality control analysis, that is, the timing when the quality control analysis has not been executed due to the deferment elapses). As a result, the calculation result for the general sample analyzed within the period in which the execution timing of the quality control analysis is deferred and the calculation result for the general sample analyzed outside the period are distinguished.

The output part 90 reads from the storage part 80 the result of the concentration calculated by the quantitation part 70, and outputs the result by displaying it on the display input part 30 or prints it out on a paper. At this time, the calculation result to which the flag is attached is output so as to be distinguishable from other calculation results by, for example, adding an underline, a mark, a comment, or using a bold font. In Fig. 3, an underline is attached to the numerical value of the density of the item a.

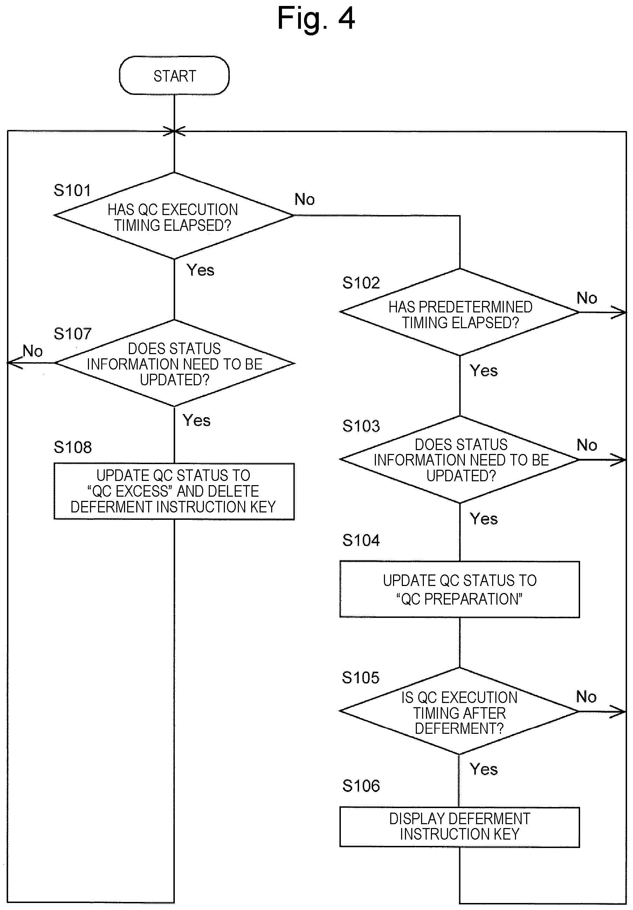

An example of the operation of the automatic analysis system according to the present embodiment will be described with reference to the flowcharts of Figs. 4 to 7. Figs. 4 to 7 are flowcharts illustrating procedures of processing of the display control part 50, the deferment control part 60, the quantitation part 70, and the output part 90, respectively. In the present embodiment, it is assumed that the first quality control analysis is executed when the analysis device 10 is started, and the flowcharts of Figs. 4 to 7 are started from the date and time when the first quality control analysis ends. Hereinafter, the quality control analysis will be abbreviated as QC.

As illustrated in Fig. 4, in step S101, the display control part 50 determines whether or not the latest (scheduled to be performed next) QC execution timing has elapsed. The QC execution timing is set in advance by a setting stored in the timing setting storage part 40. If the latest QC execution timing has not elapsed, the process proceeds to step S102, and if the latest QC execution timing has elapsed, the process proceeds to step S107.

When it is determined in step S101 that the latest QC execution timing has not elapsed, the display control part 50 determines in step S102 whether or not a predetermined timing before the QC execution timing has elapsed. In a case where the predetermined timing has elapsed, the process proceeds to step S103. In a case where the timing has not elapsed, the process returns to step S101.

In step S103, the display control part 50 determines whether or not the status information needs to be updated (whether or not the status information is "QC good"). In a case where the update is necessary, the display control part 50 updates the status information to "QC preparation" in step S104. When the update is not necessary (when the status information has already been updated to "QC preparation"), the process directly returns to step S101.

When the status information is updated in step S104, the display control part 50 determines in step S105 whether or not the latest QC execution timing is the execution timing after the deferment. In a case where the latest QC execution timing is not the execution timing after the deferment, the display control part 50 causes the display input part 30 to display (enable) the deferment instruction key A in step S106. Then, the process returns to step S101. When the latest QC execution timing is the execution timing after the deferment, the process returns to step S101 without performing step S106. As a result, the QC execution timing after the deferment is not deferred again.

When it is determined in step S101 that the latest QC execution timing has elapsed, the display control part 50 determines in step S107 whether or not the status information needs to be updated (whether or not the status information is "QC preparation"). In a case where the status information needs to be updated, the display control part 50 updates the status information to "QC excess" in step S108, and deletes the deferment instruction key from the display input part 30. Then, the process returns to step S101. When the status information does not need to be updated (when the status information has already been updated to "QC excess"), the process directly returns to step S101.

The above processing is repeatedly executed during the system operation, which manages the QC execution timing. In a case where the QC is normally performed when the user performs a predetermined operation (for example, a sample for QC is set in the analysis device 10, and an instruction to perform QC is input to the analysis device 10) and there is no problem in the result, the status information is reset to "QC good ", the QC execution timing is updated to the next QC execution timing, and the operations of steps S101 to S108 are repeated.

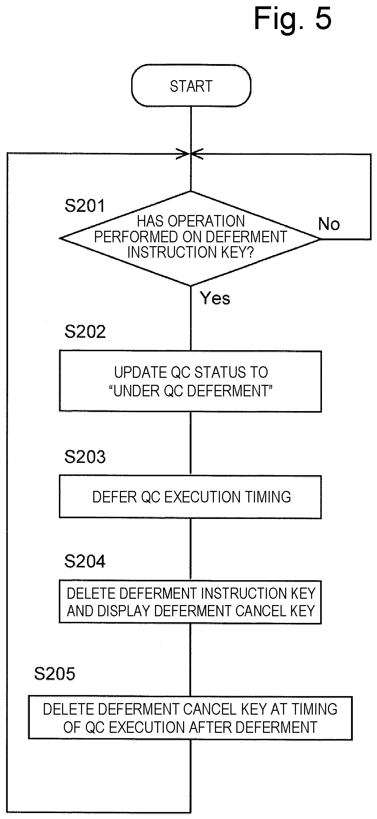

After the deferment instruction key is displayed in step S106, in a case where a predetermined operation is performed on the deferment instruction key, processing by the deferment control part 60 is performed. As illustrated in Fig. 5, in step S201, the deferment control part 60 determines whether or not a predetermined operation has been performed on the deferment instruction key displayed on the display input part 30. In a case where the predetermined operation has not been performed, the process returns to step S201. In a case where the predetermined operation has been performed, the deferment control part 60 updates the status information to "under QC deferment" in step S202, and defers the QC execution timing of the QC scheduled to be performed subsequently to the execution timing of the QC scheduled to be performed further subsequently, in step S203. Furthermore, in step S204, the deferment instruction key is deleted (disabled), and the deferment cancel key is displayed on the display input part 30. Steps S202, S203, and S204 may be performed simultaneously in any order. Then, in step S205, the deferment control part 60 deletes the deferment cancel key at the timing when the QC is performed after the deferment. After step S205, the process returns to step S201, and the subsequent processing is repeated during the analysis.

According to the above processing, since the deferment instruction key is displayed on the display input part 30 at the predetermined timing before the QC execution timing, the user can defer the QC execution timing depending on the use situation of the device. In addition, since the status information displayed on the display input part 30 is updated to "QC preparation" or "QC excess" at a predetermined timing, it is possible to prompt the user to prepare QC or execute QC. Moreover, since the status information is updated to "under QC deferment" based on the predetermined operation performed on the deferment instruction key, it is possible to easily confirm that QC has been deferred. Furthermore, since the deferment cancel key is displayed when a predetermined operation is performed on the deferment instruction key, the user can set the cancellation of the deferment.

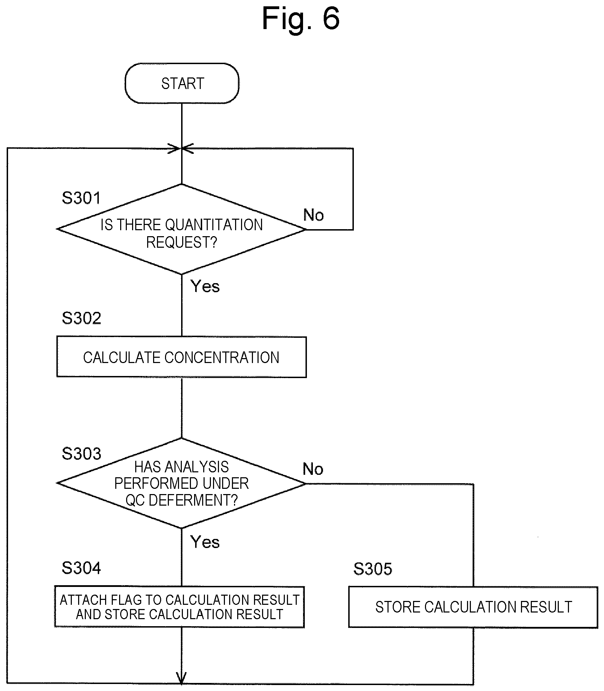

In addition, as illustrated in Fig. 6, in step S301, the quantitation part 70 determines whether or not there is a quantitation request from the analysis device 10. If there is no quantitation request, the process returns to step S301. When there is a request for quantitation, in step S302, the quantitation part 70 calculates the concentration of the inspection item component in the general sample from the measurement data measured by the analysis device 10 based on a calibration curve created in advance. Next, in step S303, the quantitation part 70 determines whether or not the general sample for which the concentration has been calculated has been analyzed within a period in which the execution timing of QC has been deferred (until the deferred QC execution timing elapses, after the preset QC execution timing, that is, the timing when the QC has not been executed due to deferment elapses). In a case where the general sample has been analyzed within the period in which the QC execution timing is deferred, in step S304, the quantitation part 70 attaches a flag to the calculation result calculated in step S302, and stores the calculation result in the storage part 80. In a case where the general sample has not been analyzed within the period in which the QC execution timing is deferred, in step S305, the quantitation part 70 does not attach a flag to the calculation result calculated in step S302, and stores the calculation result in the storage part 80. After steps S304 and S305, the process returns to step S301, and the subsequent processing is repeated.

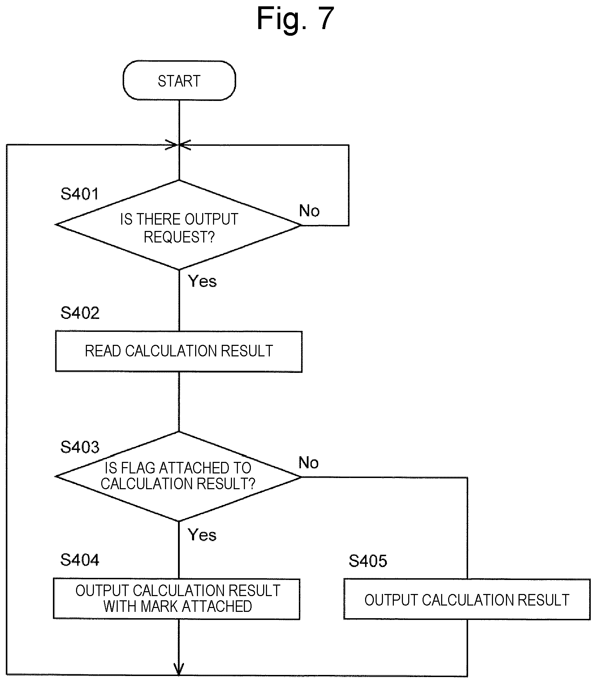

Furthermore, as illustrated in Fig. 7, in step S401, the output part 90 determines whether or not there is an output request. If there is no output request, the process returns to step S401. In a case where there is an output request, the output part 90 reads from the storage part 80 the calculation result stored by the quantitation part 70, in step S402. Next, in step S403, the output part 90 determines whether or not a flag is attached to the calculation result. In a case where a flag is attached to the calculation result, the output part 90 displays, in step S404, the calculation result on the display input part 30 in a state where a mark such as an underline, a mark, or a comment is attached. In a case where no flag is attached to the calculation result, the output part 90 displays, in step S405, the calculation result on the display input part 30 as it is. After steps S404 and S405, the process returns to step S401, and the subsequent processing is repeated.

According to the above processing, the result of the quantitative analysis can be output with the sample analyzed during deferment of the QC execution timing being distinguished from the sample analyzed otherwise.

### (Modifications)

Although the embodiment of the present invention is described with specific examples, the present invention is not limited to such an embodiment, and an appropriate modification within the scope of the present invention is possible.

In the configuration of the above embodiment, when the execution timing of the quality control analysis is deferred, the timing is deferred to the next execution timing, and the deferred execution timing is not repeatedly deferred. However, the most deferred timing may be set to the next execution timing, and the execution timing may be repeatedly deferred little by little by the next execution timing.

In the above embodiment, when the status information is updated, a pop-up screen may be displayed at the time of update so that the user can easily recognize the status information, or notification may be performed by sound or lamp.

In the configuration of the above embodiment, the execution timing of the quality control analysis is deferred by performing a predetermined operation on the deferment instruction key displayed on the display input part 30. However, a button or the like different from the display input part 30 may be enabled based on the status information displayed on the display part being updated to "QC preparation", and the execution timing of the quality control analysis may be deferred by pressing the button.

### [Modes]

A person skilled in the art can understand that the previously described illustrative embodiment is a specific example of the following modes of the present invention.

(Clause 1) An automatic analysis system according to one mode of the present invention includes:
an analysis part configured to be able to selectively perform a general sample analysis and a quality control analysis;
a display input part;
a timing setting storage part which stores a setting regarding an execution timing of the quality control analysis;
a display control part configured to display a deferment instruction key on the display input part at a predetermined timing before the execution timing determined based on the setting stored in the timing setting storage part; and
a deferment control part configured to defer the execution timing based on a fact that a predetermined operation is performed on the deferment instruction key.

According to this mode of present invention, in an automatic analysis device configured to perform a quality control analysis at a preset timing, an analysis control appropriate to given situation can be performed.

(Clause 2) The automatic analysis system according to Clause 1, may further include:
a storage part configured to store a result; and
an output part configured to output the result stored in the storage part with a result of a general sample analysis executed within a period in which the execution timing is deferred being distinguished from a result of a general sample analysis executed outside the period.

According to this mode of present invention, it is possible to easily determine which one of the analysis results is the result of the sample analyzed during the deferment of the quality control analysis. When there is a problem with the quality control analysis after the deferment, it is sufficient to adopt only the result of the sample analyzed during the deferment, which facilitates the analysis result confirmation work.

(Clause 3) In the automatic analysis system according to Clause 1 or 2,
discrimination information for discriminating an analysis status of the quality control analysis may be displayed on the display input part, and
the deferment control part may be configured to set the discrimination information during deferment based on a fact that a predetermined operation is performed on the deferment instruction key.

This makes it easier for the user to recognize that the timing of the quality control analysis has been deferred.

(Clause 4) In the automatic analysis system according to Clause 1 or 2,
the deferment control part may be configured to display a deferment cancel key on the display input part based on a fact that a predetermined operation is performed on the deferment instruction key.

According to this mode of present invention, during the deferment of the quality control analysis, the deferment can be canceled by the determination of the user.

(Clause 5) A display processing device for an automatic analysis system according to one mode of the present invention is a display processing device for an automatic analysis system equipped with an analysis part configured to be able to selectively perform a general sample analysis and a quality control analysis, including:
a display input part;
a timing setting storage part which stores a setting regarding an execution timing of the quality control analysis;
a display control part configured to display a deferment instruction key on the display input part at a predetermined timing before the execution timing determined based on the setting stored in the timing setting storage part; and
a deferment control part configured to defer the execution timing based on a fact that a predetermined operation is performed on the deferment instruction key.

According to this mode of present invention, in an automatic analysis device configured to perform a quality control analysis at a preset timing, an analysis control appropriate to given situation can be performed.

### REFERENCE SIGNS LIST

- 10: Analysis Device
- 20: Display Processing Device
- 30: Display Input Part
- 40: Timing Setting Storage Part
- 50: Display Control Part
- 60: Deferment Control Part
- 70: Quantitation Part
- 80: Storage Part
- 90: Output Part

## Claims

1. An automatic analysis system comprising:
an analysis part configured to be able to selectively perform a general sample analysis and a quality control analysis;
a display input part;
a timing setting storage part which stores a setting regarding an execution timing of the quality control analysis;
a display control part configured to display a deferment instruction key on the display input part at a predetermined timing before the execution timing determined based on the setting stored in the timing setting storage part; and
a deferment control part configured to defer the execution timing based on a fact that a predetermined operation is performed on the deferment instruction key.

2. The automatic analysis system according to claim 1, further comprising:
a storage part configured to store a result; and
an output part configured to output the result stored in the storage part with a result of a general sample analysis executed within a period in which the execution timing is deferred being distinguished from a result of a general sample analysis executed outside the period.

3. The automatic analysis system according to claim 1, wherein
discrimination information for discriminating an analysis status of the quality control analysis is displayed on the display input part, and
the deferment control part is configured to set the discrimination information during deferment based on a fact that a predetermined operation is performed on the deferment instruction key.

4. The automatic analysis system according to claim 1, wherein
the deferment control part is configured to display a deferment cancel key on the display input part based on a fact that a predetermined operation is performed on the deferment instruction key.

5. A display processing device for an automatic analysis system equipped with an analysis part configured to be able to selectively perform a general sample analysis and a quality control analysis, comprising:
a display input part;
a timing setting storage part which stores a setting regarding an execution timing of the quality control analysis;
a display control part configured to display a deferment instruction key on the display input part at a predetermined timing before the execution timing determined based on the setting stored in the timing setting storage part; and
a deferment control part configured to defer the execution timing based on a fact that a predetermined operation is performed on the deferment instruction key.
